# EUROPEAN PATENT SPECIFICATION

(11) **EP 1 956 973 B1**
(45) Date of publication and mention of the grant of the patent: **13.09.2017**
(21) Application number: 06821260.4
(22) Date of filing: 30.10.2006
(51) Int. Cl.: A61B 5/0404, H01R 13/52

(54) **ELECTRO-MECHANICAL CONNECTOR FOR THIN MEDICAL MONITORING PATCH**
ELEKTROMECHANISCHER STECKVERBINDER FÜR EINE DÜNNE MEDIZINISCHE ÜBERWACHUNGSEINRICHTUNG
CONNECTEUR ELECTROMECANIQUE POUR TIMBRE DE SURVEILLANCE MEDICALE MINCE

(30) Priority: 30.11.2005 US 741492 P
(43) Date of publication of application: 20.08.2008
(73) Proprietor: Koninklijke Philips N.V., 5656 AE Eindhoven (NL)
(72) Inventor: CROSS, Brett, Seattle, WA 98119 (US); HUGH, Steven, C., Bellevue, WA 98007 (US); SOLOSKO, Thomas, A., Issaquah, WA 98029 (US)
(74) Representative: de Haan, Poul Erik
(86) International application number: PCT/IB2006/054019
(87) International publication number: WO 2007/063436

(56) References cited:
- WO-A-2004/057704
- US-A- 5 249 576
- US-A- 5 458 124
- US-A1- 2003 216 662
- US-A1- 2004 077 954
- US-B1- 6 435 882
- US-B1- 6 456 872
- US-B1- 6 603 995

## Description

The following relates to the medical arts. It finds particular application in conjunction with medical monitoring devices and will be described with particular reference thereto. It will be appreciated that the following is also applicable to other medical and non medical devices in medical and non medical fields such as athletic monitoring, animal, child monitoring, electrical stimulation, medication delivery, and the like, in a variety of applications.

In many biomedical applications, monitoring and therapy devices are attached to the patient's skin to observe and monitor patient conditions such as health, blood flow, heart rhythm, blood oxygen levels, administer therapy as required, and the like. Examples of monitoring and therapy devices include the electrocardiograph to monitor ECG, external defibrillators, pacing devices, transcutaneous nerve stimulation devices, and transdermal drug delivery systems. In some applications, such as monitoring and therapy, devices are attached for extended periods of time and must be removed prior to showering, or when changing clothes. Many of the monitoring devices are large and typically are worn on a belt with wires attached to skin-mounted, disposable electrodes. The larger devices are uncomfortable to carry. Movement of the wires, monitoring device, or electrodes due to the attachment of wires may create artifacts in the monitored waveform.

Another approach is to use smaller external devices which are typically held in place with medical grade tape completely covering the device. Such method of skin attachment provides a secure and water-resistant adhesion to the skin, but does not allow the skin to breath or move under the device. Body moisture accumulates in occluded areas which aggravates the skin. Skin held rigidly under a device is affected by movements of the device. This creates artifact in the monitored waveforms. Rigidly held skin may also become irritated, especially at the tape-to-skin boundary. In addition, the constant pressure of the device against the skin may also cause depressions in the skin, which become irritating.

US2004/0077954 A1 discloses a monitor with cardiac and movement sensors that are responsive to a user's heart beat and a user's movement. The monitor is disc-shaped and its rear comprises a recessed clip which is removably attachable to an electrical contact of a conventional ECG electrode.

US6456872 B1 discloses a Holter-type apparatus for the recording of physiological signals indicative of cardiac activity. The apparatus has a base unit formed of a flexible sheet carrying the electrodes collecting the physiological signals and the conductive connection elements connected to the electrodes. The base unit has a central area receiving a recording case and which contains contact areas forming the proximal terminations of the respective conductive connection elements. The recording case is equipped to be fastened to the base unit central area and to have electrode contacts that make electrical contact with contact areas of the base unit. The base unit also can carry a battery to supply power to the recording case. The base unit is advantageously made of a sheet of flexible printed circuit material carrying a conducting pattern forming the aforementioned electrodes, conductive connection elements and contact areas.

US6603995 B1 discloses a portable ECG monitoring apparatus comprising a sensor device detachable from a monitoring device 2. The sensor device includes ECG sensors attached via cabling to a connector. The sensor device and monitoring device are connected by attaching the connector to the monitoring device, which provides a watertight seal for the apparatus and prevents access to any internal components in the monitoring device.

US5458124A discloses a wireless transmitter module for use in a system for monitoring at least one physiological condition of a subject. The transmitter module includes a housing and an electrode patch preferably having three closely spaced electrodes provided on a first surface thereof. The first surface of the patch is coated with a non-allergenic adhesive in regions surrounding the electrodes, whereby the patch is securable to the subject by the adhesive to permit sensing of a physiological signal of the subject by the electrodes. Additionally, the module has snaps for detachably connecting the electrode patch to the exterior of the housing, the snaps being operable to effect electrical communication of the electrodes with the circuitry when the electrode patch is connected to the housing, whereby the entirety of the transmitter module is supported by the electrode patch when the electrode patch is connected to the housing and the first surface is adhered to the subject.

US 5249576 A relates to a pulse oximeter probe, in which a sensor has a pin and socket mechanical configuration. A rectangular element consisting of anisotropically conducting elastomer which provides electrical conductivity only in the thickness dimension thereof is included in the sensor connector.

US 6435882 B1 mentions an anisotropic electrically conductive film, and US 2003/0216662 Almentions a Z-axis-only conductive adhesive.

The present invention provides new and improved apparatuses and methods which overcome the above -referenced problems and others. The invention is defined by the independent claims. Features of further advantageous embodiments of the invention are defined in the dependent claims.

In embodiments of the modular device, the medical device includes device electrical contacts. A patch includes a conductive first layer, which is in direct electrical communication with skin. A patch connector, which includes electrical contacts in electrical communication with the first conductive layer, establishes an electrical communication path between the skin and medical device contacts and affixes the medical device in close proximity to the patch.

In embodiments of the method of connecting a medical device to a subject base surface, a patch, which includes a conductive first layer, is disposed in direct electrical communication with the base surface. The patch is electro mechanically connected to a medical device connection interface which includes a first connector and device contacts.

In accordance with another aspect, a connector is disclosed which comprises a clip. The clip includes a first surface; a second surface opposite the first surface; extending portions disposed on the first surface; and a band of elastomeric material which extends from about the first surface to about the second surface to electrically contact device contacts of a monitoring device on the first surface and patch contacts on the second surface when the extending portions engage with a case of the medical device.

Still further advantages and benefits of the present invention will become apparent to those of ordinary skill in the art upon reading and understanding the following detailed description of the preferred embodiments.

The invention may take form in various components and arrangements of components, and in various steps and arrangements of steps. The drawings are only for purposes of illustrating the preferred embodiments and are not to be construed as limiting the invention.
FIGURE 1 is a diagrammatic illustration of a modular medical device;
FIGURE 2 is an expanded view of a patch assembly;
FIGURE 3A is a perspective view of one face of a connector which includes pins;
FIGURE 3B is a perspective view of an opposite face of the connector of FIGURE 3A;
FIGURE 4 is a perspective view of a portion of a mechanical interface of connector of FIGURE 3A;
FIGURE 5 is an expanded view of the monitoring device and the patch assembly;
FIGURE 6 is an expanded view of a patch assembly which includes an electronic board;
FIGURE 7 is a view of the monitoring device and the patch which are connected via a single piece of Z-axis conductive pressure sensitive adhesive;
FIGURE 8 is an expanded view of a patch assembly which includes a clip;
FIGURE 9A is an expanded view of a clip assembly;
FIGURE 9B is a diagrammatic illustration of the assembled clip of FIGURE 9A;
FIGURES 10A and 10B are diagrammatic illustrations of an assembly of the patch assembly of FIGURE 8 and a monitoring device;
FIGURES 11A, 11B and 11C are diagrammatic illustrations of the monitoring device which snaps completely into the clip;
FIGURE 12 is an expanded view of an assembly of the patch assembly, in which a clip is inserted through the patch layers, and a monitoring device;
FIGURE 13 is an expanded view of an assembly of a patch and a monitoring device;
FIGURE 14 is a diagrammatic illustration of one side of a clip which includes a single silicon band;
FIGURE 15 is an expanded view of an assembly of a monitoring device, patch and clip of FIGURE 14;
FIGURE 16A is a perspective view of one face of a clip;
FIGURE 16B is a perspective view of an opposite face of the clip of FIGURE 16A;
FIGURE 17A is a perspective view of one side of a clip;
FIGURE 17B is a perspective view of an opposite side of the clip of FIGURE 17A;
FIGURE 18A is a perspective view of a portion of a clip;
FIGURE 18B is a perspective view of the clip of FIGURE 18A with overmolded inserts;
FIGURE 19 is a diagrammatic illustration of the monitoring device which includes reusable conductive posts;
FIGURE 20 is an expanded view of an assembly of a patch and a monitoring device of FIGURE 19;
FIGURE 21 is an expanded view of the clip molded directly onto the circuit layer;
FIGURE 22 is a perspective view of the patch of FIGURE 21 with a patch side printed circuit;
FIGURE 23 is a perspective view of the patch of FIGURE 21 with a clip side printed contact pads;
FIGURE 24 is a perspective view of the clip and the patch of FIGURE 21; and
FIGURE 25 is a perspective view of the assembled clip and patch of FIGURE 21.

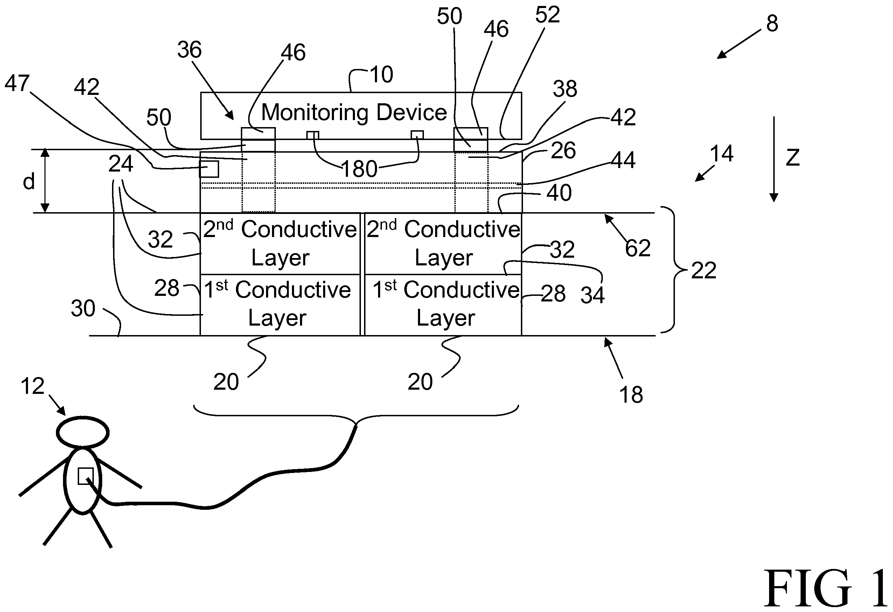

With reference to FIGURE 1, a modular device **8** includes a monitoring or therapy or other medical device **10** which is attached to a patient **12** via a patch assembly **14.** The patch assembly **14** provides a communication path between the monitoring device **10** and a base surface or skin **18** of the patient **12** in one or more areas **20**. More specifically, the patch assembly **14** includes a patch or patch laminate **22** including a plurality of layers **24** and an electromechanical connector or connection interface **26.** In one embodiment, a thickness **d** of the electromechanical interface **26** is less than 10mm. Examples of medical devices **10** are cardiac ECG event monitors, ECG Holter recorders, and cardiac emergency alerting devices.

A first conductive material layer or pieces of first conductive material or first electrodes **28,** such as conductive hydrogel, are disposed at a proximate or first surface **30** of the patch assembly **14** to make direct contact with the skin **18**. A second layer or electrodes or patch circuit layer **32** is disposed proximately to a top surface **34** of the first conductive hydrogel layer **28.** In one example, the second electrodes **32** are constructed from silver/silver chloride (Ag/AgCl) or from any other suitable material. In one embodiment, the electrodes **32** are disposed in communication with the skin **18** to measure the voltage difference between two or more locations on the body. Although only two patch contacts **32** are illustrated, it is contemplated that a number of contacts may be greater than two.

The monitoring device **10** includes a connection interface **36.** The patch connector **26** is disposed proximately to the monitoring device **10** at a connector first or top layer or surface **38** and to the patch assembly **14** at a connector second or bottom layer or surface **40.** Ionic conduction from the skin **18** passes through the first conductive or hydrogel material layer **28**, changes to electronic conduction in the circuit layer **32** and as such is passed to the monitoring or therapy device **10** via electrical contacts or connection interface **42** of the electromechanical connector **26**. As discussed in detail below, in one embodiment, the patch connector **26** is a stand alone connector which is disposed between the monitoring device **10** and the patch laminate **22**. In one embodiment, the patch connector **26** includes connection interfaces disposed throughout the patch layers **24**. In one embodiment, the connector **26** includes a rigid base layer **44** and/or a mechanical member or members **47** to establish a rigid mechanical connection between the patch **22** and the monitoring device **10.**

The first conductive hydrogel layer **28** improves the electrical conductivity between the electrodes **32** and the skin **18.** Typical components of a conductive hydrogel include water, which acts as the solvent, water-soluble monomers, which crosslink to give structure to the gel and which may also provide skin adhesion, humectant materials which reduce the dryout characteristics of the hydrogel material, and electrolytes or salts such as sodium chloride or potassium chloride dissolved in water, which provide and facilitate ionic movement and conductivity. One advantage of hydrogel materials over other conductive electrolytes is that the hydrogel material can be removed cleanly from the skin without leaving a residue

The electromechanical connector **26** provides the electrical connections between the patch circuit layer **32,** which is in electrical contact with the skin **18** via the first conductive layer **28,** and the monitoring or therapy device **10.** The electrical connections may either be low impedance connections, such as a pin-to-metal pad connection, or high impedance connections, such as a higher-impedance conductive silicone to metal pad connection. The electromechanical connector **26** also provides the mechanical connection to the monitoring device **10** via a monitoring, device connection interface or second or device connector **46.** The methods of mechanical connection as well as the type of electrical connection, either high or low impedance, play a role in reducing artifact in the monitored signal as described below.

Electrical artifacts include artifacts due to common mode voltages and electrostatic charges that affect the skin, electrode patch and monitoring device. Motion artifacts include voltages produced by the skin, fat and muscle during skin stretching under the electrodes, movement of the electrode or sensor on the skin, intermittent electrode or sensor contact with the skin, wire movement, and movement of the monitoring or therapy device which translates to movement of electrodes or sensors on the skin. In addition, light sensors are also subject to artifact from external light sources.

Rigid mechanical connections can drive the artifact frequency band higher, while, looser, floppy mechanical connections can dampen artifacts and drive the artifacts to a lower frequency. Rigid patch materials may hold the skin tighter and reduce the amplitude of artifacts caused by skin and muscle stretching. Decoupling the electrodes from each other and from the monitoring or therapy device may decrease the affect of device movement on the electrodes.

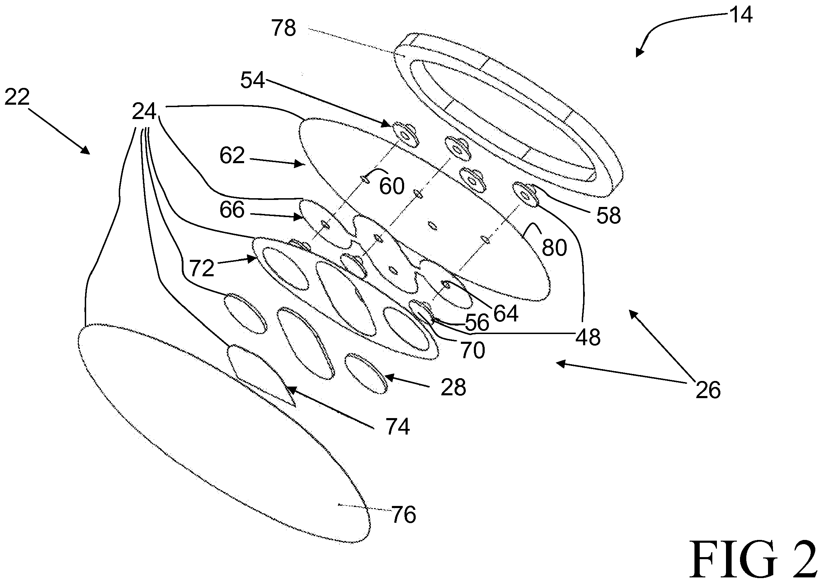

With continuing reference to FIGURE 1 and further reference to FIGURE 2, the second electrodes **32** are omitted. The patch connector **26** provides low impedance electrical connection of the patch assembly **14** to the medical device **10.** More specifically, the electrical contacts **42** of the patch connector **26** include snaps **48** which connect to device contacts **50** which are disposed about a bottom surface **52** of the monitoring device **10.** The snaps **48** provide electrical and mechanical connection of the patch assembly **14** to the monitoring device **10.** More specifically, each snap **48** includes a snap top **54** and a snap eyelet **56.** Each snap top **54** includes a snap post **58** which is inserted into respective matching snap receptacle (not shown) in the monitoring device **10** to hold the snaps **48** rigidly in place. Three, four, five, six or more snaps **48** form a stabilizing plane to hold the monitoring device **10** rigidly against the patch assembly **14** and distally from the base surface or skin **18.**

Each snap top **54** connects with respective snap eyelet **56** through corresponding openings **60** in a retention seal layer **62,** and openings **64** in a snap support layer **66** to establish electrical contact. The retention seal layer **62** protects the hydrogel layer **28** from outside water entry; while the snap sealing layer **66** prevents the snaps **48** from tearing out of the patch assembly **14.** The snap sealing layer **66** is constructed from polyester or other appropriate stiff supporting material. In one embodiment, the snap eyelets **56** are constructed from a conductive material. In another embodiment, each snap eyelet **56** is coated with a conductive material such as silver/silver chloride to provide low offset and low noise body signal (ECG or other) measurements. A first face or side **70** of each snap eyelet **56** makes contact with the first conductive layer **28** such as pieces of hydrogel material which make contact with the base surface or skin **18**. The first pieces of hydrogel material **28** are disposed in a hydrocolloid or frame layer **72**

A non-conductive liquid-proof sealing layer **78** between the monitoring device **10** and the patch assembly **14** surrounds the snaps **48** to provide additional protection for the snaps **48** from outside liquids such as shower water. In one embodiment, the sealing layer **78** includes a single, compressible, elastomer gasket that is bonded to a top surface **80** of the retention seal layer **62** which compresses when the monitoring device **10** snaps onto the snaps **48.** In one embodiment, the height of the uncompressed gasket is greater than the snap posts **58**. The gasket compresses and creates the seal as the snaps mate with the respective mating receptacles. Individual discrete seals may be used around each snap **48** in place of the single elastomer gasket.

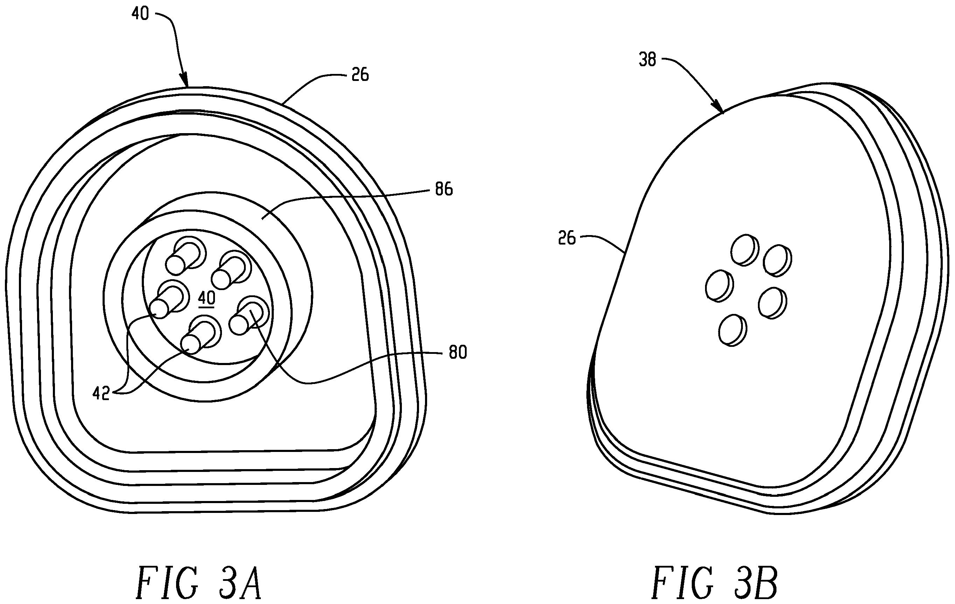

With continuing reference to FIGURE 1 and further reference to FIGURES 3A and 3B, the patch assembly **14** in this embodiment includes the patch connector **26** which is a molded connector which includes individual connector contacts **42** such as individual pogo-style spring-loaded pins **80** to provide a low impedance connection. The pins **80** are post-inserted or insert molded into the connector through the top or first surface **38** of the connector **26** (as seen in FIGURE 3B) to allow the spring-loaded end of each pin **80** to extend through the second or bottom surface **40** of the connector **26** into a sealing boss **86.** The sealing boss **86** is drafted inwards to provide mechanical locking feature for mechanical connection with the monitoring device **10.** More specifically, a top surface of the sealing boss **86** which is distal from the bottom surface **40** of the connector **26** is larger than a rear surface of the sealing boss **86** which is proximate to the bottom surface **40** of the connector **26.**

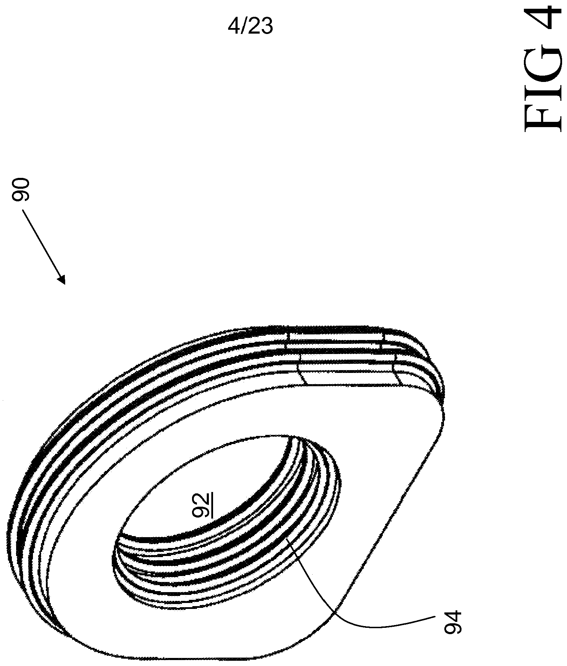

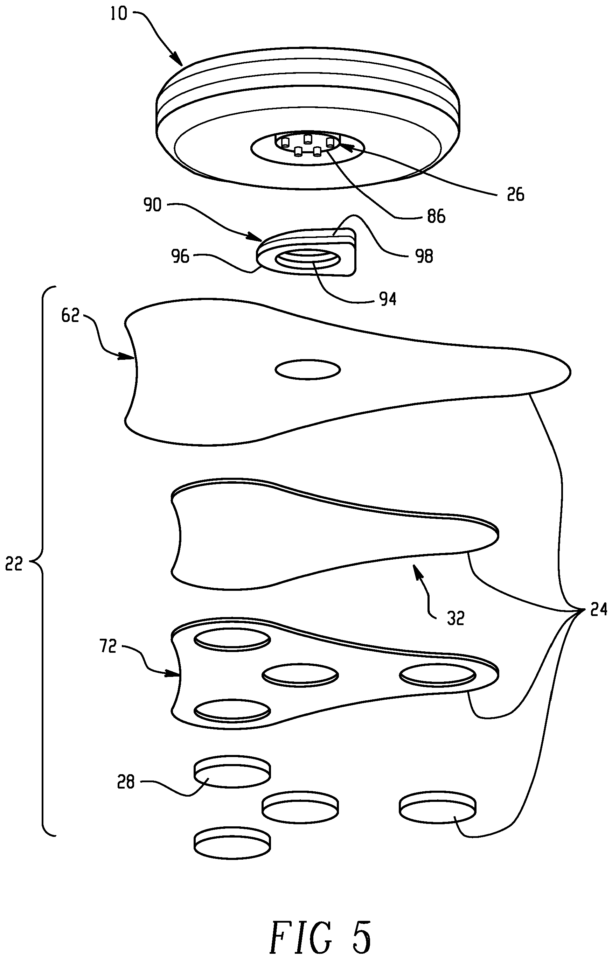

With continuing reference to FIGURES 3A and 3B and further reference to FIGURES 4 and 5, a non-conductive elastomeric sealing boot **90**, which includes an opening **92** with ribbed walls **94** in a central portion, is connected to the connector **26.** More specifically, when the connector **26** and the sealing boot **90** are mated, the rigid sealing boss **86** pushes through the ribbed walls **94** of the center opening **92** of the sealing boot **90,** compressing the elastomer material and creating radial force which holds the patch **22** via the boot **90** onto the connector **26.** To reinforce, the inward draft on the walls of the sealing boss **86** prevents the sealing boot **90** from sliding off. Since the compressed elastomer wants to relax, the effect of the inward draft causes the boot **90** to slide inward, towards the inside of the connector **26,** further trapping and holding the boot **90** and the patch **22** against the connector **26.** Outside walls **96** of the sealing boot **90** include ribs **98** which compress and deflect against the inner walls of the connector **26** to form a seal against outside moisture entry into the boss **86** of connector **26**.

Once the sealing boot **90** is mated with the connector **26,** the spring-loaded pins **80** contact metal pads or traces provided on the patch circuit layer **32** of the patch **22.** The pads or traces lead to the first individual conductive pieces **28** and carry signals from the first pieces **28** to the monitoring device **10** and from the monitoring device **10** to the first pieces **28.** For example, the pads and traces may be printed using silver, silver/silver chloride, or conductive carbon ink on a polyester or PVDF substrate. As another example, the pads and traces may be plated copper traces on a flexible polyester or Kapton substrate. As yet another example, the pads and traces may be part of a printed circuit board.

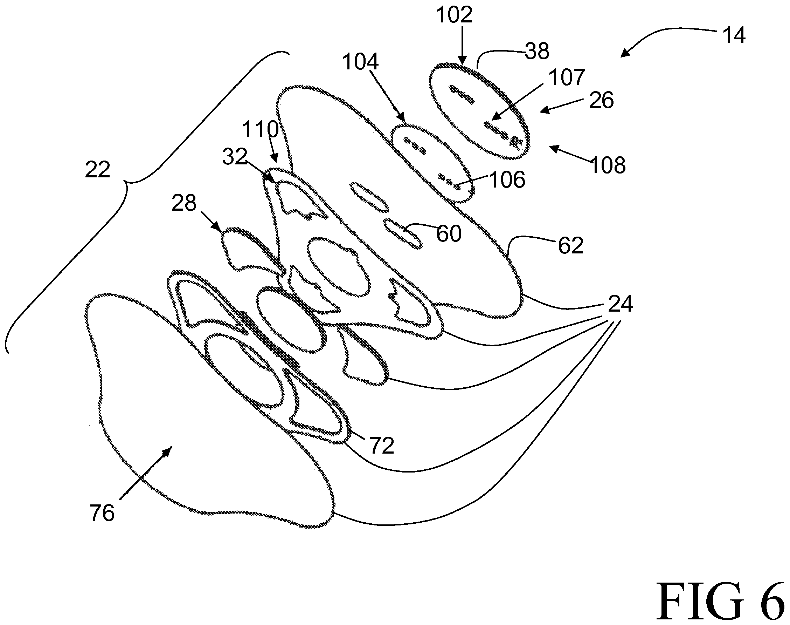

With reference again to FIGURE 1 and further reference to FIGURE 6, the electrical contacts **42** of the patch connector **26** of this embodiment include a connector printed circuit layer or board **102**, which is bonded to the retention seal layer **62** via a diecut piece of a non-conductive pressure sensitive adhesive layer (PSA) **104**. The retention seal layer **62** and pressure sensitive adhesive layer **104** include respective openings **60**, **106** to be filled with conductive epoxy **107** which electrically connects the patch circuit layer **32** to conductive pads **108** disposed on the printed circuit board **102.** More specifically, during the assembly, the pressure sensitive adhesive layer **104** is aligned over printed circuit pads **110** on the patch circuit layer **32,** which for example is, a printed polyester layer, and adhered to the retention seal layer **62.** Each of the openings **106** in the PSA layer **104** is filled with conductive epoxy. The connector printed circuit board **102** is aligned with the PSA layer **104**. The entire patch assembly **14** is placed in a heated chamber to hasten the cure of the conductive epoxy. Alternatively, since the conductive epoxy is trapped between the circuit board **102** and the patch circuit layer **32,** the conductive epoxy may be allowed to cure at room temperature as the patch assembly **14** is packaged and shipped.

In one embodiment, the top surface **38** of the connector **26,** includes a multipin straight connector or right angle header with or without a housing snap which enables the electromechanical attachment of the patch assembly **14** to the monitoring device **10.** In one embodiment, the sealing gasket is bonded to the top surface **38** of the connector **26.** Upon insertion of the connector **26** into the monitoring device **10,** the gasket compresses to protect the connector pins from liquid entry.

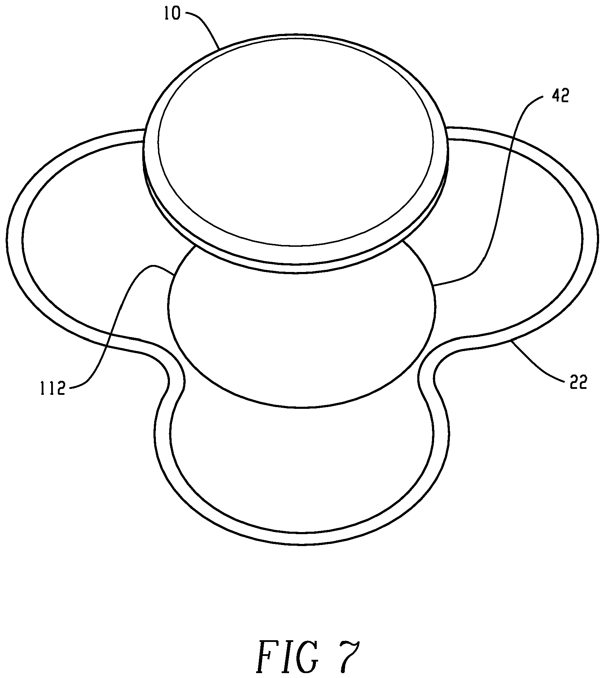

With continuing reference to FIGURE 1 and further reference to FIGURE 7, the patch connector electrical connection interface **42** includes a single piece or layer **112** of Z-axis conductive pressure sensitive adhesive (PSA), which directly electrically connects the patch electrodes **32** to the device contacts **50** on the bottom surface **52** of the monitoring device **10.** To establish a reliable connection, the Z-axis conductive PSA is pressed between the top surface **38** of the patch assembly **14** and the bottom surface **52** of the monitoring device **10** with a sufficient amount of pressure for a sufficient amount of time, which is specific to the individual material. For example, a pressure of 30 PSI which is applied for 5 seconds may be sufficient for 3M 9703™, but may not be sufficient for a different PSA. Many conductive PSA materials are not suitable for use alone as structural PSA layers. A strengthening, non-conductive PSA layer is applied around such conductive PSA to improve strength and support, and to bring the Z-axis conductive PSA into compression which provides a more consistent electrical connection.

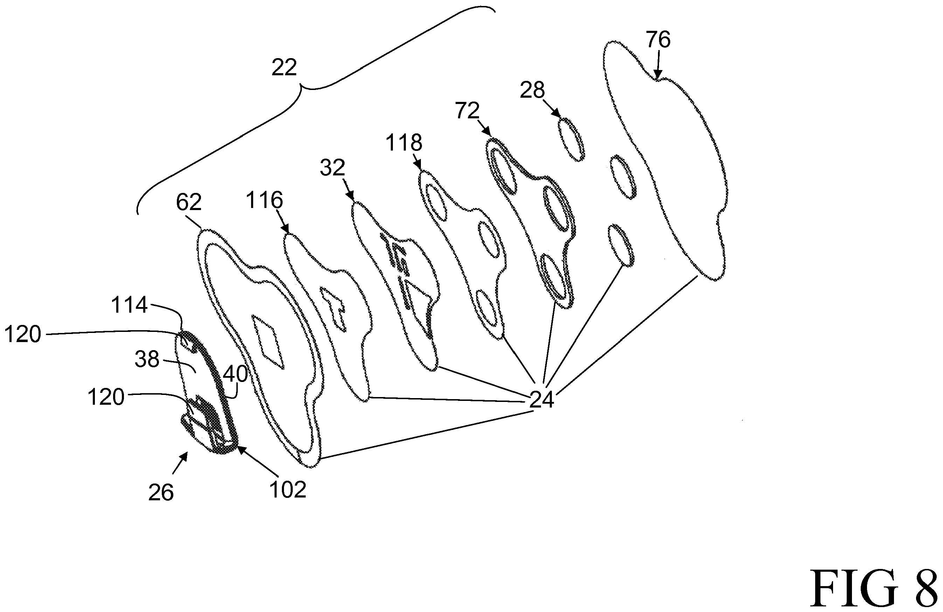

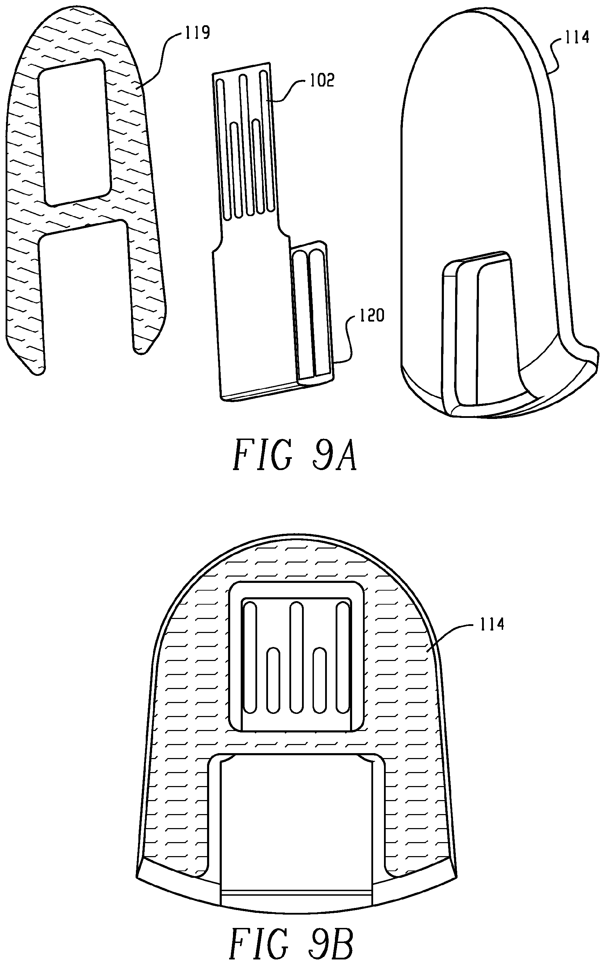

With continuing reference to FIGURE 1 and further reference to FIGURE 8, 9A and 9B, the patch assembly connector **26** of this embodiment includes a clip **114.** The clip **114** includes the top surface **38** proximate the monitoring device **10,** bottom surface **40** proximate the patch **22** and the connector printed circuit layer **102,** such as a flexible circuit layer, which mates with the patch circuit layer **32,** which, in this embodiment, is a flexible circuit layer disposed between first and second dielectric layers **116, 118.** The connector flexible circuit layer **102** is aligned so that its traces mate with respective traces of the patch circuit layer **32.** To improve electrical connection between the two sets of traces, a low-impedance Z-axis conductive adhesive is applied to the connector flexible circuit **102** which bonds the connector printed circuit **102** to the patch circuit **32** and electrically connects the traces. The clip **114** includes a non-conductive layer of pressure sensitive adhesive **119** which bonds the clip **114** to the patch **22** mechanically and structurally. The clip non-conductive PSA layer **119** provides the liquid-proof seal between the clip **114** and the patch 22.

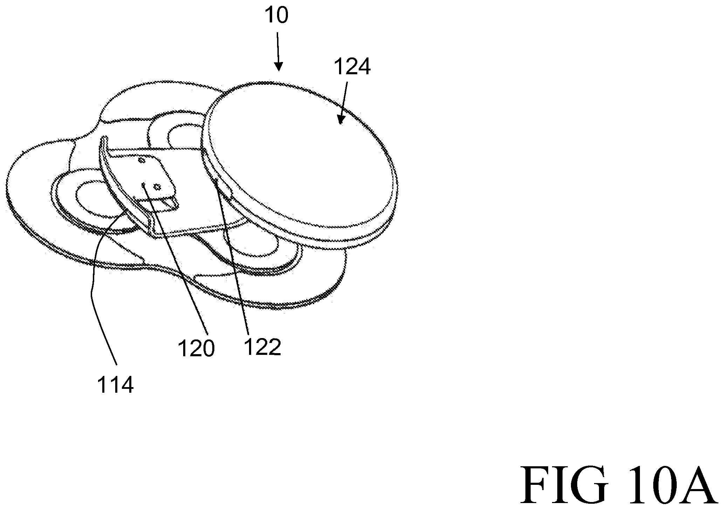

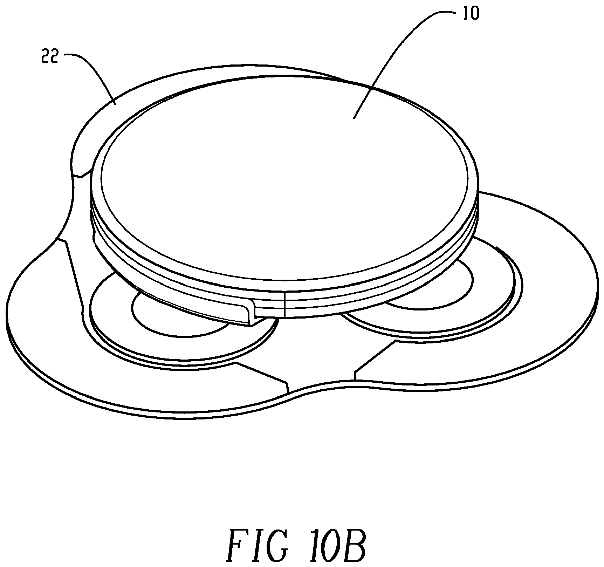

With continuing reference to FIGURES 1 and 8 and further reference to FIGURES 10A and 10B, a tongue or extending portion **120** of the clip **114** is inserted into a slot **122** of a case **124** the monitoring device **10.** Once inside the monitoring device **10,** the traces on the bottom surface of the clip tongue **120** mate with the device contacts **50** in the monitoring device **10.** This completes the electrical circuit between the patch traces and the monitoring device **10.**

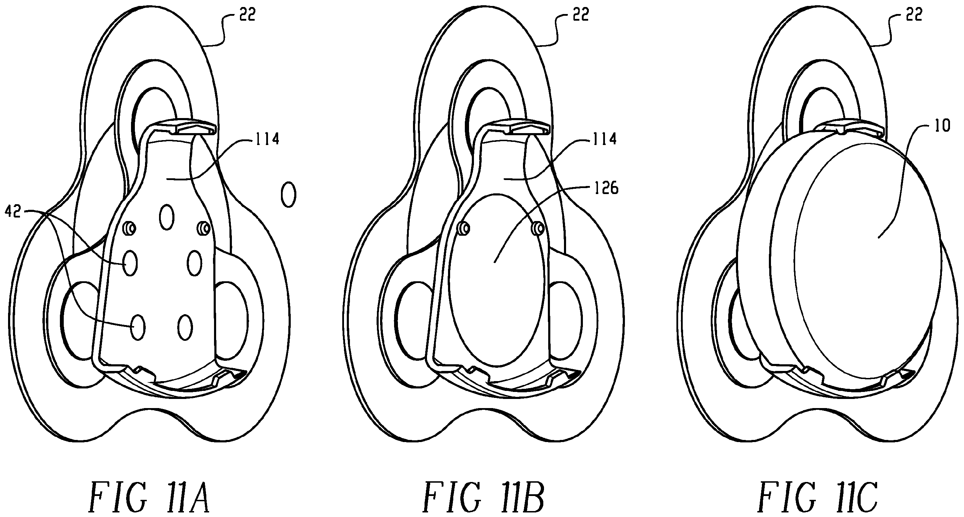

With continuing reference to FIGURES 1 and 8 and further reference to FIGURES 11A, 11B and 11C, the monitoring device **10** snaps completely into the clip **114,** which is, for example, a snap action clip. As a result of such mechanical connection, the device contacts **50,** such as pins and leaf springs, on the bottom surface **52** of the monitoring device **10** connect electrically with corresponding patch metal contacts **42,** i.e., gold-plating over nickel-plated copper pins, or the connector printed circuit board **102** of the clip **114** through a single piece of thin, Z-axis conductive elastomer sheet **126.** The elastomer sheet **126** compresses as the clip **114** snaps onto the monitoring device **10.** Once compressed, the conductive particles, i.e. carbon particles or fibers, are pressed against conductive surfaces of the clip and monitoring device making a connection. In addition, since the elastomer sheet is conductive in the Z-axis only, such elastomer sheet forms a seal against outside moisture entry when compressed. The patch **22** is attached to the clip **114** with one or more pieces of pressure sensitive adhesive. A conductive adhesive, such as 3M 9703™, can be used to make the electrical connection between the patch traces and the metal contacts in the clip **114.** A piece of non-conductive structural adhesive may be used around the conductive PSA layer to improve the strength and reliability of the bond between the patch 22 and clip **114.**

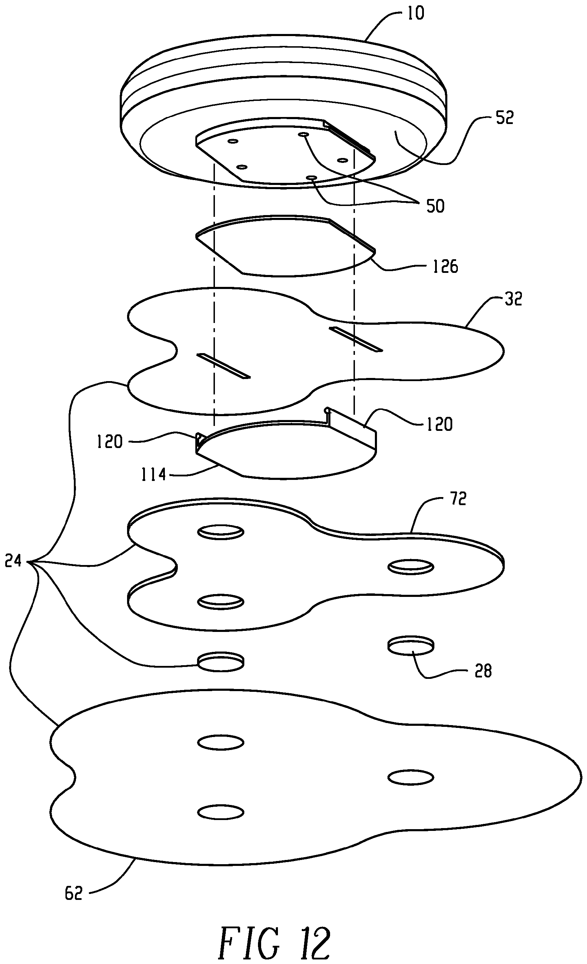

With reference to FIGURE 12, to eliminate the conductive adhesive between the clip **114** and the patch **22,** the clip **114** includes one or more extending portions **120** which are inserted through the patch circuit layer **32.** The clip **114** is held in place by the frame layer **72.** The patch connector electrical connection interface **42** includes the layer **126** of a Z-axis conductive elastomer material which provides electrical connection between the monitoring device **10** and the patch circuit layer **32.** The patch traces are directly exposed to the Z-axis conductive silicone layer **126,** which electrically connects the patch traces to the device contacts **50** on the bottom surface **52** of the monitoring device **10.** Such direct connection is more reliable and robust, since every additional electrical interface increases the risk for the patch failure. By passing under and through the patch circuit **32** and mechanically by snap action connecting with the monitor **10,** the clip **114** provides a rigid support surface against which the monitoring device **10** may press into the patch traces. The rigid surface provides a more consistent pressure between the monitoring device and patch contacts.

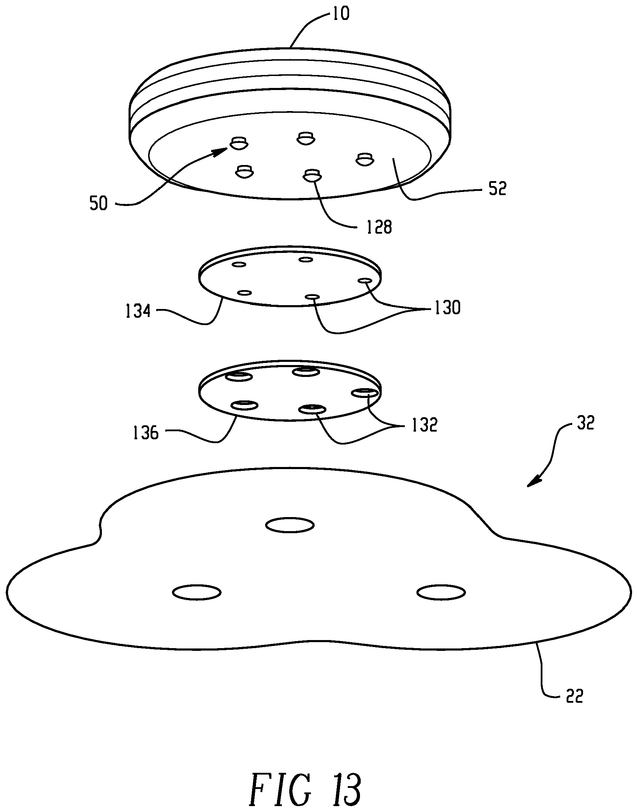

With reference to FIGURE 13, the device contacts **50** of the monitoring device **10** include pins which make direct contact with the patch circuit layer **32.** The monitoring device pins **50** each includes a shoulder **128,** which tapers to a point near the end. The pins **50** press through or make openings **130, 132** in respective first or top and second or bottom layers **134, 136** of a non-conductive elastomer. The top layer openings **130** have smaller diameter than the bottom layer openings **132.** Further, the diameter of each top layer opening **130** is smaller than the dimensional measurements of the shoulder **128** of the pins **50**. The top layer opening **130** traps respective shoulder **128** of each pin **50** once the pins are pressed through the openings **130, 132** and force the pins **50** to contact exposed pads or traces on the patch circuit layer **32.**

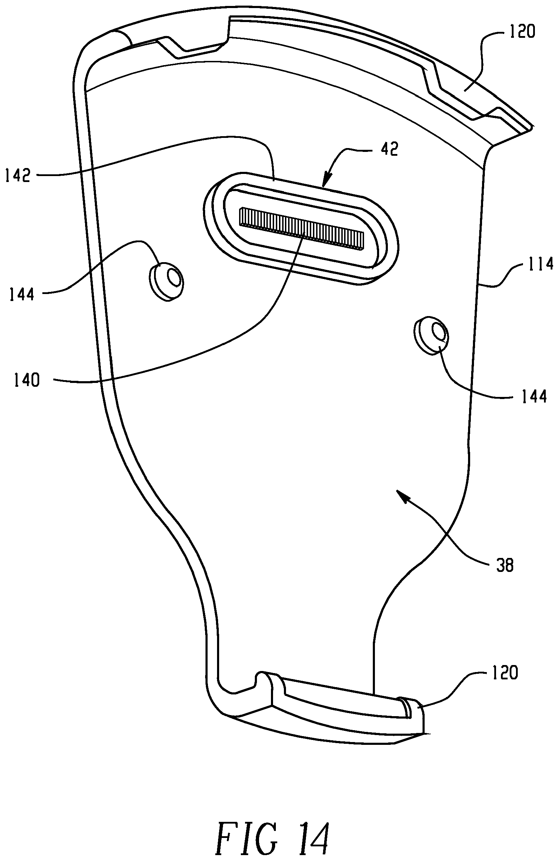

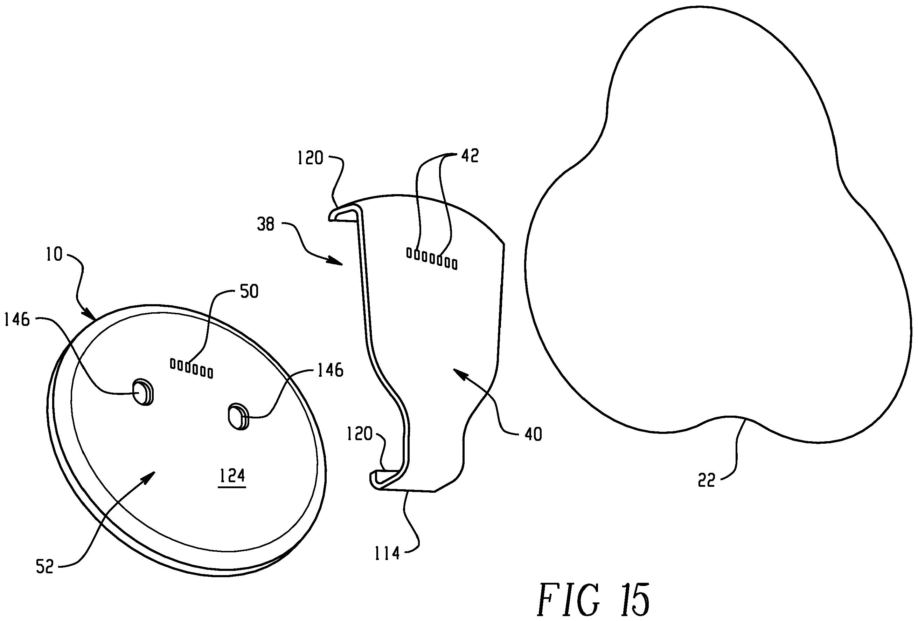

With reference again to FIGURE 1 and further reference to FIGURES 14 and 15, the patch connector electrical connection interface **42** includes a single band of higher-impedance Z-axis conductive elastomer **140,** such as silicone, surrounded by a single, conductive or non-conductive elastic seal **142.** When compressed, the conductive silicone **140** provides electrical connection between the device contacts **50** on the bottom surface **52** of the monitoring device **10** and conductive traces on the patch **22**. For example, the conductive silicone **140** can be inserted or over-molded to pass completely through the clip **114** and make contact with the monitoring device **10** on the clip top surface **38** and the conductive traces or pads of the patch **22** on the clip bottom surface **40.** E.g., no additional clip contacts are required. Since the conductive silicone **140** electrically connects the patch traces to the device contacts **50** on the monitoring device **10,** non-conductive PSA may be used to bond the patch layers to the clip **114.** Although, the device contacts **50** of the monitoring device **10** are shown to be flush with the bottom surface **52** of the monitoring device **10,** it is contemplated that the device contacts **50** of the monitoring device **10** can extend beyond the bottom surface **52** of the monitoring device **10** or be recessed into the bottom surface **52** of the monitoring device **10.** The clip extensions **120** releasably engage the case **124** of the medical device **10** to hold the medical device **10** and the patch **22** together.

To ensure enough compression of the conductive silicone, the patch traces may be backed up with another material, such as a thicker, firmer material, e.g. 0.032 inch-thick polyethylene foam.

The elastic seal **142** surrounds the single piece or array of conductive silicone element(s) and, when compressed against the base of the monitoring device, prevents liquid entry into the contact area.

First alignment structures or means **144** disposed on the first clip surface **38** mate with second alignment structures or means **146** disposed on the bottom surface **52** of the monitoring device **10** so that the connector electrical contacts **42** are aligned with the device contacts **50.** Although pin-type alignment structures are shown, other alignment structures may be employed, such as mated notches and tongues along the periphery of the clip surfaces. Also, the medical device **10** and clip **114** may be asymmetrically shaped such that device **10** insertion can be accomplished in only one orientation into clip **114.**

In one embodiment, individual conductive silicone contacts are overmolded or post-inserted into the rigid clip **114** in place of a single piece of z-axis conductive elastomer **140.** The individual contacts can also be co-molded into a single connector or subassembly part using a non-conductive elastomer or polymer to bridge the gap between each contact.

To improve electrical conductivity between the individual conductive elastic contacts and the patch, the bottom surface of the contacts may be printed or otherwise coated with conductive ink. In addition, a piece of Z-axis conductive adhesive may be laminated between the patch and the clip contacts, inside a window in the surrounding structural pressure sensitive adhesive

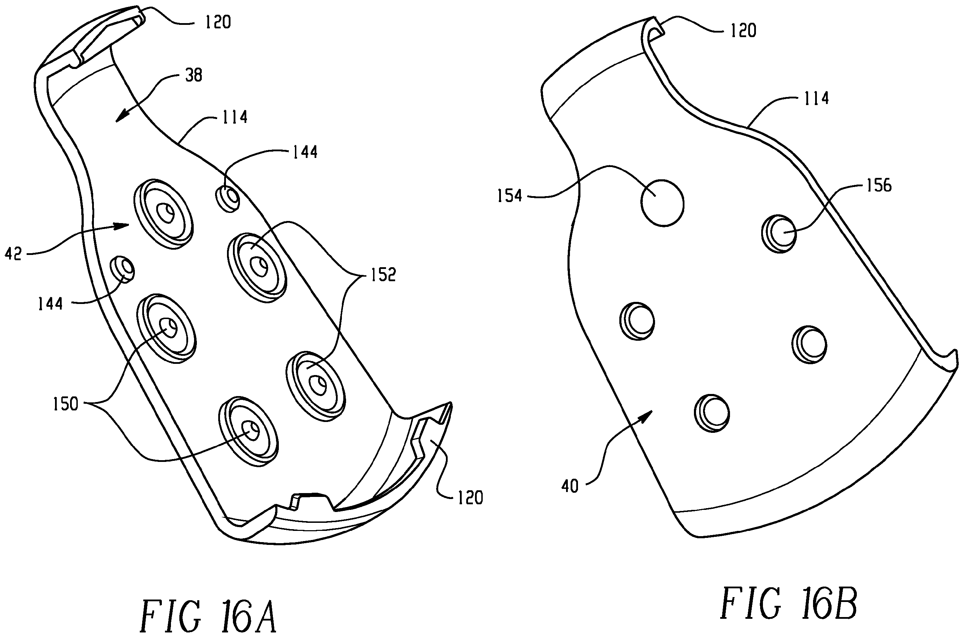

With continuing reference to FIGURE 1 and further reference to FIGURES 16A and 16B, similar to the embodiments described above, the rigid clip **114** mechanically attaches the patch **22** to the monitoring device **10.** The clip electrical contacts **42** include individual pieces or contacts **150** of the conductive elastomer, such as conductive silicone, which electrically connect the patch traces to the device contacts **50** on the bottom surface **52** of the monitoring device **10**. Each individual conductive silicone piece **150** is surrounded by an elastomer seal **152.** Such individual seals allow the individual pieces of silicon to maintain isolation from each other even if one seal fails.

In one embodiment, the elastomer contacts **150** are molded or inserted partway into the clip **114.** The clip electrical contacts **42** further include silver/silver chloride (Ag/AgCl) plated plugs **154** which are insert-molded or post-inserted or applied into the bottom surface **40** of the clip **114** through openings **156** to make contact with each respective conductive elastomer contact **150** and the first hydrogel layer **28** of the patch 22.

As another example, the plug **154** is constructed from a conductive metal or plastic, such as a glass-fiber reinforced conductive acrylonitrile butadiene styrene (ABS), that is molded into shape. It is contemplated that the plug may or may not include a flange for touching the gel, and a post for press-fitting into the clip. The formed or molded plug is plated with the Ag/AgCl before or after molding.

As another example, the plug **154** can be die cut from a thin sheet of metal or conductive polymer, and then plated with the Ag/AgCl.

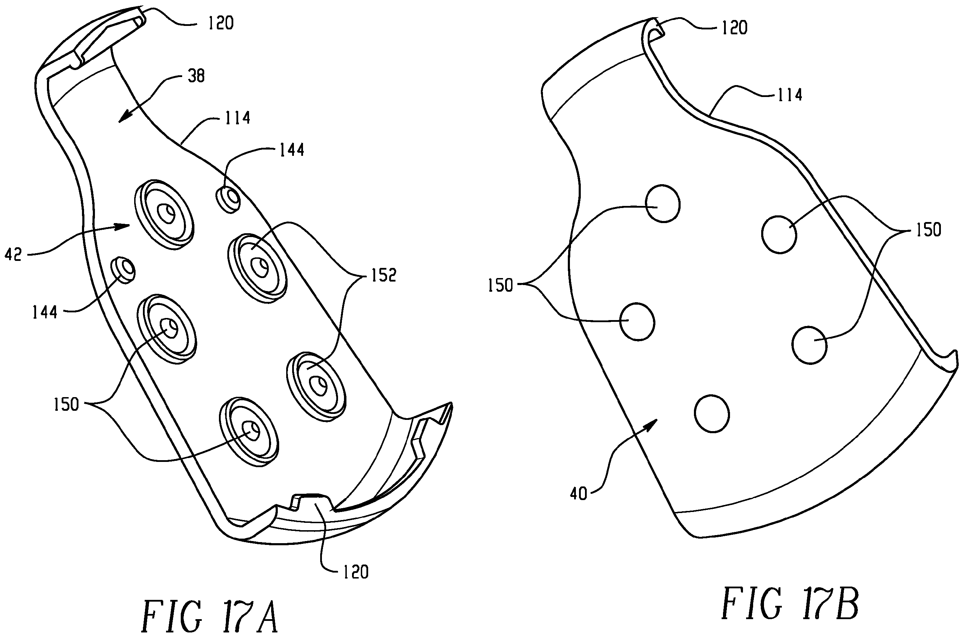

With continuing reference to FIGURE 1 and further reference to FIGURES 17A and 17B, the clip **114** is similar to the clip of the embodiments described above, except the Ag/AgCl plugs **154** are omitted. The pieces **150** of the conductive elastomer material are overmolded through the entire thickness of the clip **114** so that the pieces **150** extend out or are flush with both the top and bottom surfaces **38, 40** of the clip **114.** The exposed bottom surface **40** makes contact with metal traces or contacts of the patch **22,** while the top surface **38** is exposed for contact with the device contacts **50** of the monitoring device **10**.

In one embodiment, to create a half-cell reaction, the surface of each piece **150** of the elastomer material, such as silicone, is pad printed or screen-printed with a Ag/AgCl ink to make contact with the first conductive hydrogel layer **28** to form a half-cell reaction. To adequately adhere to the surface of the silicone pieces, the ink may be formulated in a silicone base.

In another embodiment, to create a half-cell reaction, the pieces of the conductive silicone are loaded with Ag/AgCl particles. E.g., the Ag/AgCl particles may be the conductive material in the silicone. If the loading is of high concentration, the Ag/AgCl particles in the cured silicone will form an adequate half-cell reaction as at the contact with the hydrogel.

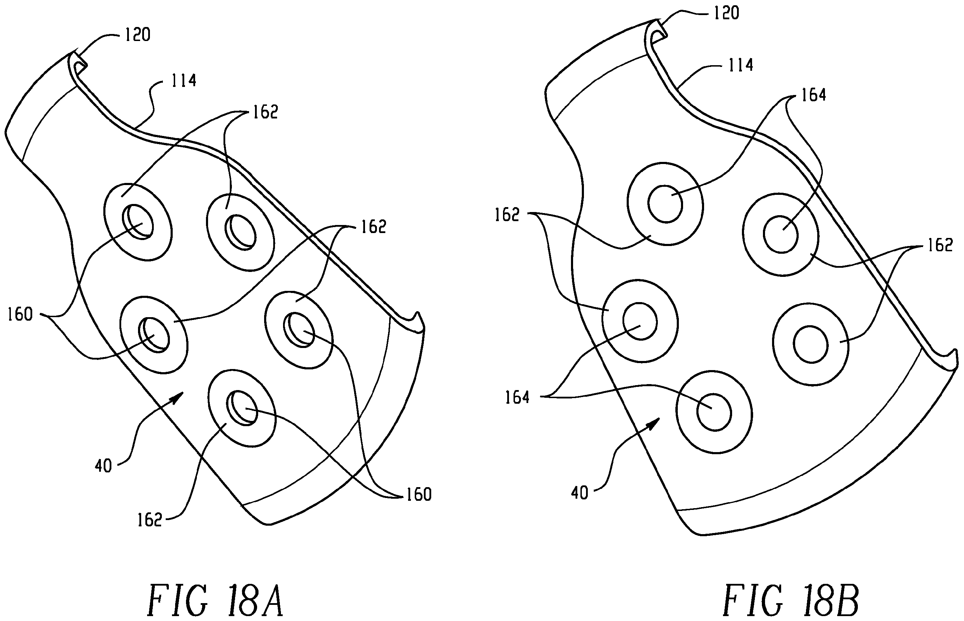

With continuing reference to FIGURE 1 and further reference to FIGURES 18A and 18B, the clip **114** includes clip openings **160.** The clip electrical contacts **42** include rings **162** of Ag/AgCl which are pad or screen printed directly to the bottom surface **40** of the clip **114** to surround each clip opening **160.** The clip electrical contacts **42** further include conductive silicone **164** which is overmolded completely through the openings **160** to overlap a portion of the printed Ag/AgCl rings **162** on the bottom surface **40** which contacts the patch **22.** Upon contact with the first hydrogel layer **28,** the rings **162** of Ag/AgCl create the half-cell reaction. The conductive silicone **164,** which overlaps each printed ring **162** conducts the body signals to the contacts of the monitoring device **10.**

In one embodiment, a vacuum is used to draw the Ag/AgCl ink inside the clip openings **160.** In this design, the conductive silicone does not need to flow all the way through the clip opening **160** since the contact with the Ag/AgCl is made inside the clip opening **160.**

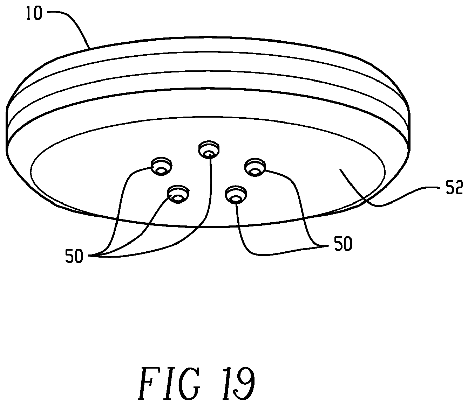

With continuing reference to FIGURE 1 and further reference to FIGURE 19, the device contacts **50** include reusable conductive posts which are molded or inserted into the bottom housing of the monitoring device **10** to make direct contact with the hydrogel layer **28** of the patch assembly **14**. For example, such posts are formed of metal or molded of conductive polymer. After forming, the posts are plated with Ag/AgCl before being inserted into the housing of the monitoring device **10.** Since the posts can be cleaned between each application of the patch **22** to the patient, the Ag/AgCl coating should be thick and robust enough to withstand multiple cleanings and patch applications. Alternatively, the posts may be sintered out of Ag/AgCl. This eliminates plating the posts afterwards, and ensures that Ag/AgCl is not taken off the posts. The patch **22** may be mechanically attached to the monitoring device **10** in several ways.

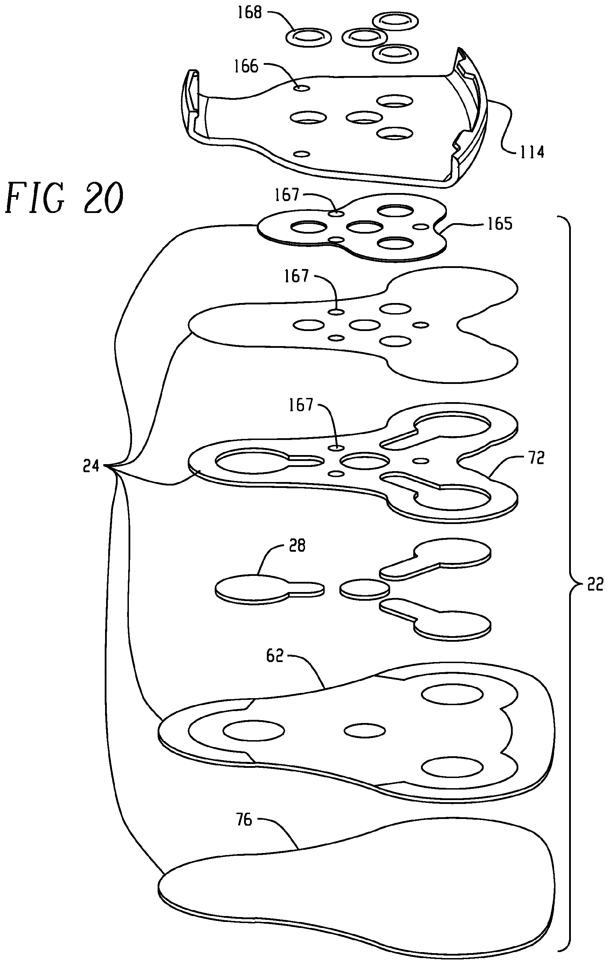

With continuing reference to FIGURES 1 and 19 and further reference to FIGURE 20, the patch **22** is attached to the monitoring device **10** with the clip **114,** which is bonded to the patch **22** with a non-conductive PSA layer **165**. Through the matching openings **166, 167** correspondingly provided in the clip **114** and the patch layers **24,** the posts or device contacts **50** pass through the patch layers **24** and touch the first hydrogel layer **28** directly. Individual protective O-rings **168** are provided to seal and protect each post from liquid entry during bathing or showering.

As another example, the patch **22** can be attached to the monitoring device **10** via a non-conductive PSA layer, or a multi-layer PSA laminate. Openings are provided in the PSA layer to allow the Ag/AgCl posts to pass through and contact the first hydrogel layer **28**. The PSA layer holds the patch **22** to the monitoring device **10** and seals around and between the individual posts. To ensure a consistent seal, a thicker PSA layer, or a thin (20 mil or 32-mil) PE or PU foam with adhesive on both sides may be used in place of the thick PSA. Being compressible, the foam compensates well for variations of Ag/AgCl post protrusion distances. In one embodiment, different adhesives on each side of the foam or PSA layer are used. More specifically, an aggressive PSA layer is used on the patch side and a less aggressive, easier to peal PSA layer, is used on the monitoring device side for the PSA material to come cleanly off of the monitoring device so that the monitoring device **10** can quickly be cleaned and prepared for use with a new patch.

The direct connection of the silver/silver chloride contacts with the hydrogel layer **28** eliminates conductive traces on the patch **22** and minimizes the number of connections required to make connection between the monitoring device and the base layer, improving reliability and potentially decreasing noise artifact. Without traces, the patch circuit layer **32** can become relatively inexpensive to manufacture. This also increases the material choices available for the circuit layer. For example, thinner polyester or PVDF films may be used if no printing is required. As the thinner films are used, the patch **22** becomes increasingly flexible and comfortable.

As mentioned above, Ag/AgCl is desirable as the hydrogel contact material for monitoring electrodes due to the stability of the resulting half-cell reaction.

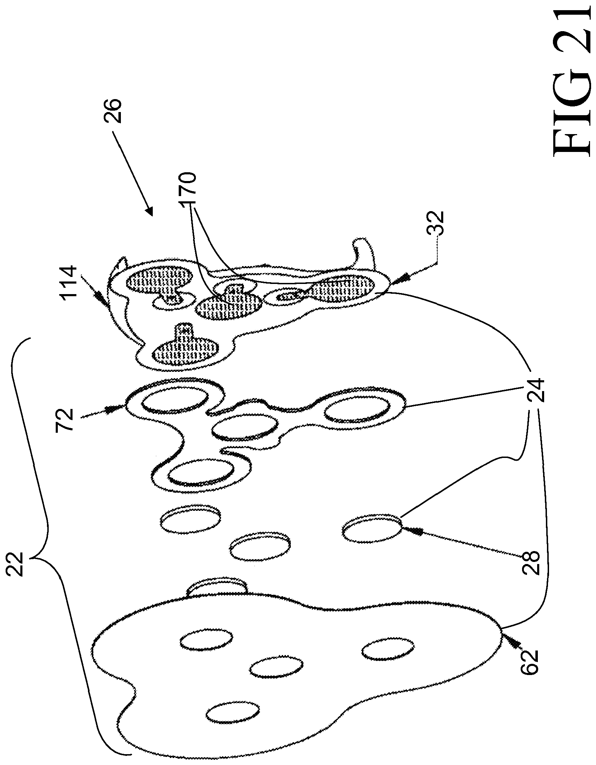

With continuing reference to FIGURE 1 and further reference to FIGURE 21, the clip **114** is molded over a part of the patch circuit layer **32** via a technique similar to in-mold decorating techniques. More specifically, in-mold decorating techniques place a pre-formed printed polyester film into the injection mold against the inside surface of the mold. The molten polymer is then shot against the film and cooled. Once cooled, the polyester film is inseparable from the polymer. In one embodiment, the patch circuit layer **32** is a thin, printed polyester layer, suitable for placement in an injection-molding tool. As one example, the patch circuit **32** may be preformed into a shape. As another example, the patch circuit **32** may nor be preformed into a shape. After insertion in the tool, the clip material is injected and cooled against its surface. This creates a strong mechanical bond between the patch circuit **32** and clip **114.** The clip **114** includes the openings (not shown), which are positioned to communicate with the contact pads (not shown) in the circuit layer **32.** The clip electrical connection interface **42** includes conductive silicone which is subsequently overmolded into the clip openings to form a robust electrical connection with the patch circuit **32.** Non-conductive elastomeric rings may be molded or bonded around each conductive silicone contact for sealing against the surface of the monitoring device **10.**

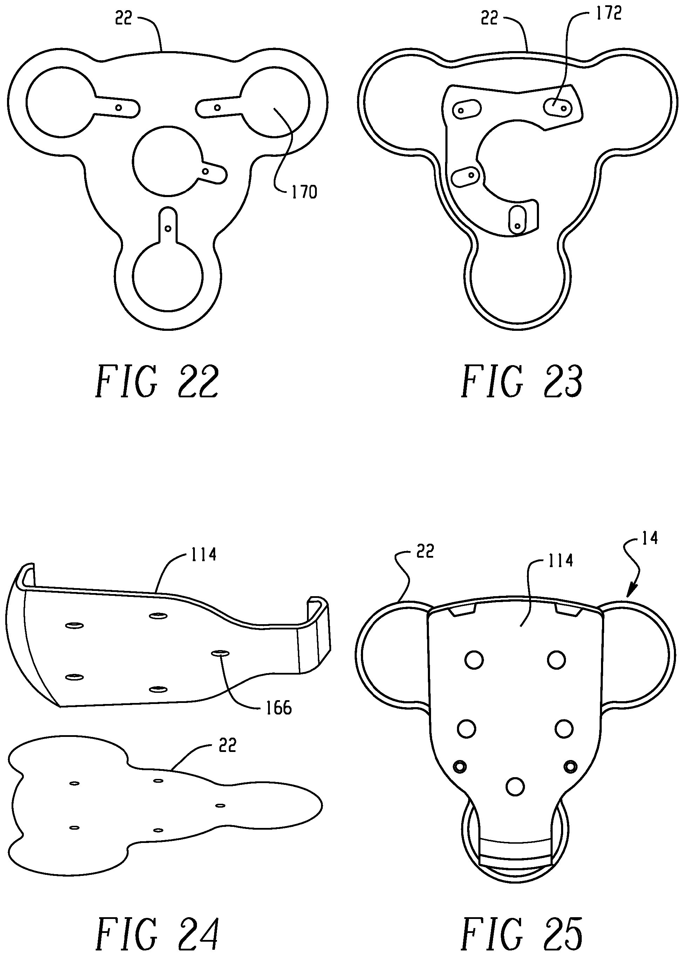

With continuing reference to FIGURE 21 and further reference to FIGURES 22, 23, 24 and 25, the clip **114** and circuit sub-assembly is bonded to the patch **22**. The foam layer or support layer **72** is coated on both sides with non-conductive PSA material. The PSA material connects and seals the patch circuit layer **32** and clip **114** to the foam layer **72.** The foam layer **72** is attached to the hydrogel pieces **28** and retention seal **62**.

FIGURE 22 shows the patch circuit **32** proximate to the patch layers **24,** with a patch side printed circuit **170**.

FIGURE 23 shows the patch circuit **32** proximate to the clip **114** with clip side printed contact pads **172.**

FIGURE 24 shows the clip **114** and the patch **22.** The clip **114** includes clip openings **166** for the monitoring device posts or overmolding of conductive silicone contacts.

In one embodiment, the monitoring device **10** includes sensors **180** which detect body motion such as respirations, footfalls, heart beats and CPR compressions.

In this manner, by connecting the monitoring device to the medical patch via the thin clip helps to detect motion artifact in the monitored signal, which allows the signal processor in the monitoring device to compensate accordingly.

With reference again to FIGURE 1, in one embodiment, the patch connector **26** is a low-profile electro-mechanical connection that creates a rigid patch area only in the center of the patch, leaving the outer areas of the patch flexible to bend and stretch with the skin. This minimizes the affect of monitor movement on the patch, thus reducing noise artifact due to monitor movement.

Other embodiments exist which include combinations of embodiments mentioned herein, combinations of low and higher-impedance contact mechanisms on the same connector, and embodiments that many include one, multiple or no seals.

The invention described above can be applied to other fields where electronic devices are attached to be held firmly to the skin to monitor physiologic signals or responses such as cardiac stress testing. One example is the athletic training field where electronic devices are worn to monitor performance. Other examples include child monitoring, such as for SIDS where a monitor is attached to the child for long periods of time to monitor cardiac and respiration activity, biosignal monitoring to monitor the health of the animals, and transdermal drug delivery systems which monitor certain patient parameters to determine when additional drug is required, how much is required, and the effects of drug dosage.

The methods and apparatuses described above provide a low-profile method of mechanically attaching the monitoring device to a thin, flexible patch. The thin, low-profile connections allow the monitoring device to become closely coupled to the bio-electrode patch enabling motion artifact detection by the monitoring device. When the electro-mechanical connection is thin enough, motion sensors in the monitoring device (accelerometers or piezo-electric sensors) can be designed to detect patient movements including footfalls, respirations and heartbeats.

The methods and apparatuses described above prevent the monitoring device from directly contacting the skin.

The methods and apparatuses described above include a low-profile, wire-free method of electrically connecting the patch electrodes to the monitoring device. Eliminating the wires can reduce electrical artifact in the bioelectric signal.

The methods and apparatuses described above include a method for creating a liquid-proof seal around each patch contact, or around the group of contacts, when the patch is connected to the monitoring device. Sealing between electrodes ensures that shorting does not occur during a shower or spill. The methods and apparatuses described can also be implemented without the seals.

The methods and apparatuses described above include a rigid central portion. Since the monitoring/therapy device is a relatively large mass attached to the skin, small movements or rotations in the device can create large disturbances in the monitored signal. A rigid portion in the center of the connector stabilizes the monitoring/therapy device and helps reduce motion artifact by preventing excess movement and rotation of the device during patient movement. When only the center is rigid, this connection scheme allows the edges of the patch to conform to body contours.

The methods and apparatuses described above are user friendly in that they may allow single step connections (both the mechanical and electrical connections are accomplished at the same time with the same user action). They can be performed with a single hand, and they do not require or transmit heavy forces to the body. They also allow attaching the monitoring device to the patch before attaching the patch to the skin.

The methods and apparatuses described above take advantage of the high-impedance patient monitoring electronics in the monitoring device. When impedances of the monitoring electronics are very high (i.e. Giga-ohm range), connector embodiments can be developed which are in the 1000 - 10,000 Ohm range without significantly affecting the monitored signal. The impedance attribute of the connector can be either high - 100 - 10,000 Ohm, medium 20-200 Ohm, or low, < 20 Ohm.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. A modular device (8) for attachment to the skin (18) of a patient (12) comprising:
a medical device (10) which includes device electrical contacts (50) on a bottom surface (52) of the medical device (1);
a patch laminate (22) including a plurality of layers (24), the plurality of layers (24) comprising a conductive first layer (28) for establishing a direct contact with the skin (18);
a patch connector (26), which includes connector electrical contacts (42) in electrical communication with the first conductive layer (28) and establishes an electrical communication path between the skin (18) and medical device contacts (50) and which affixes the medical device (10) in close proximity to the patch laminate (22),
wherein the plurality of layers (24) further comprises an electrical distribution layer (32) which is electrically connected with the connector electrical contacts (42) and with each of a plurality of first electrodes defined in the first conductive layer (28), and
wherein the patch connector includes a generally rigid clip (114) including a clip first surface (38), and a clip second surface (40), the clip first surface (38) including extending portions (120) which releasably engage a case (124) of the medical device (10) to hold the connector contacts (42) and the device contacts (50) in electrical communication; the patch connector (26) further:
providing a rigid patch area in a center of the patch laminate (22), and a flexible outer area of the patch laminate (22), and
comprising a layer of a Z-axis conductive elastomer material (126) configured to be compressed as the clip (114) is snapped onto the medical device (10) and to provide an electrical connection between the medical device contacts (50) and the connector electrical contacts (42).

2. The device as set forth in claim 1, wherein the patch connector (26) further includes:
alignment means (144, 146) for indexing the connector electrical contacts (42) to match the medical device contacts (50).

3. The device as set forth in claim 1, further including:
a fluid seal (142, 152) which surrounds the connector contacts (42).

4. The device as set forth in claim 1, wherein the connector electrical contacts (42) include at least one of:
pins (80);
snaps (48);
silver/silver chloride elements (154, 162); and
electrically conductive elastic segments (150, 164).

5. The device as set forth in claim 1, wherein the connector electrical contacts (42) include:
a band (140) of a z-axis conductive elastic material in contact with the electrical distribution layer (32).

6. The device as set forth in claim 1, further comprising:
a sensor (180) which detects at least one parameter attributable to a motion of the skin.

7. The device as set forth in claim 1, wherein the clip (114) passes under the electrical distribution layer (32) and the extending portions (120) thereof are inserted through the electrical distribution layer (32) for providing a rigid support surface.

8. A method of connecting a medical device (10) to a subject base surface (18) comprising:
disposing a patch laminate (22) which includes a conductive first layer (28) in direct electrical communication with the base surface;
electromechanically connecting the patch to a medical device connection interface (36) which includes a first connector (46) and device contacts (50) via a patch connector (26) wherein the patch connector includes connector electrical contacts (42) in electrical communication with the first conductive layer (28), and a generally rigid clip (114) including a clip first surface (38), and a clip second surface (40), the clip first surface (38) including extending portions (120), wherein a rigid patch area is provided in a center of the patch laminate (22), the patch laminate (22) comprising a flexible outer area,
electrically connecting an electrical distribution layer (32) of the patch laminate (22) with the connector electrical contacts (42) and with each of a plurality of first electrodes defined in the first conductive layer (28),
the step of connecting the patch including:
releasably engaging a case (124) of the medical device (10) with the extending portions (120) of the clip first surface (38) to hold the connector contacts (42) and the device contacts (50) in electrical communication, thereby establishing an electrical communication path between the subject base surface (18) and the medical device contacts (50) and affixing the medical device (10) in close proximity to the patch laminate (22),
wherein a layer of a Z-axis conductive elastomer material (126) is compressed as the clip (114) is snapped onto the medical device (10) and provides an electrical connection between the medical device contacts (50) and the connector electrical contacts (42).

9. The method as set forth in claim 8, wherein the patch layers further include second electrodes (32) and further including:
disposing the first electrodes in direct electrical communication with the base surface and the second electrodes;
disposing the second electrodes in electrical communication with the device contacts; and
transmitting electrical signals generated in the base surface to the medical device contacts.

10. The method as set forth in claim 9, further including:
sealing the electrical communication path from the base surface to the device contacts against fluids with a sealing layer.

11. The method of claim 10, wherein the patch connector includes electrical contacts and the sealing layer includes elastic rings which each surrounds each respective individual patch electrical contact.

12. The method as set forth in claim 8, wherein said connecting step occurs after said disposing step.

## Patentansprüche

1. Modulare Vorrichtung (8) zum Anbringen an der Haut (18) eines Patienten (12), umfassend:
eine medizinische Vorrichtung (10), die elektrische Vorrichtungskontakte (50) auf einer Bodenfläche (52) der medizinischen Vorrichtung (10) umfasst;
ein Pflasterlaminat (22) umfassend eine Vielzahl von Schichten (24), wobei die Vielzahl von Schichten (24) eine leitfähige erste Schicht (28) zum Herstellen eines direkten Kontakts mit der Haut (18) umfasst;
einen Pflastersteckverbinder (26), der elektrische Steckverbinderkontakte (42) in elektrischer Kommunikation mit der ersten leitfähigen Schicht (28) umfasst und einen elektrischen Kommunikationspfad zwischen der Haut (18) und den medizinischen Vorrichtungskontakten (50) herstellt und der die medizinische Vorrichtung (10) in unmittelbarer Nähe an dem Pflasterlaminat (22) befestigt,
wobei die Vielzahl von Schichten (24) weiterhin eine elektrische Verteilungsschicht (32) umfasst, die elektrisch mit den elektrischen Steckverbinderkontakten (42) und mit jeder von einer Vielzahl von ersten Elektroden verbunden ist, die in der ersten leitfähigen Schicht (28) definiert sind, und
wobei der Pflastersteckverbinder einen im Allgemeinen starren Clip (114) mit einer ersten Clipfläche (38) und einer zweiten Clipfläche (40) umfasst, wobei die erste Clipfläche (38) hervorstehende Abschnitte (120) umfasst, die lösbar in ein Gehäuse (124) der medizinischen Vorrichtung (10) eingreifen, um die Steckverbinderkontakte (42) und die Vorrichtungskontakte (50) in elektrischer Kommunikation zu halten;
wobei der Pflastersteckverbinder (26) weiterhin:
einen starren Pflasterbereich in einer Mitte des Pflasterlaminats (22) und einen flexiblen Außenbereich des Pflasterlaminats (22) bereitstellt, und
eine Schicht aus einem leitfähigen Z-Achsen-Elastomermaterial (126) umfasst, die konfiguriert ist, um komprimiert zu werden, wenn der Clip (114) auf die medizinische Vorrichtung (10) geschnappt wird, und um eine elektrische Verbindung zwischen den medizinischen Vorrichtungskontakten (50) und den elektrischen Steckverbinderkontakten (42) bereitzustellen.

2. Vorrichtung nach Anspruch 1, wobei der Pflastersteckverbinder (26) weiterhin Folgendes umfasst:
Ausrichtungsmittel (144, 146) zum Indexieren der elektrischen Steckverbinderkontakte (42), um sie mit den medizinischen Vorrichtungskontakten (50) abzugleichen.

3. Vorrichtung nach Anspruch 1, weiterhin umfassend:
eine Fluiddichtung (142, 152), die die Steckverbinderkontakte (42) umgibt.

4. Vorrichtung nach Anspruch 1, wobei die elektrischen Steckverbinderkontakte (42) mindestens eines von Folgendem umfassen:
Stifte (80);
Druckknöpfe (48);
Silber/Silberchlorid-Elemente (154, 162); und
elektrisch leitfähige elastische Segmente (150, 164).

5. Vorrichtung nach Anspruch 1, wobei die elektrischen Steckverbinderkontakte (42) Folgendes umfassen:
ein Band (140) eines leitfähigen elastischen Z-Achsen-Materials in Kontakt mit der elektrischen Verteilungsschicht (32).

6. Vorrichtung nach Anspruch 1, weiterhin umfassend:
einen Sensor (180), der mindestens einen einer Hautbewegung zuzuschreibenden Parameter detektiert.

7. Vorrichtung nach Anspruch 1, wobei der Clip (114) unter der elektrischen Verteilungsschicht (32) hindurchgeführt wird und die hervorstehenden Abschnitte (120) hiervon durch die elektrische Verteilungsschicht (32) eingeführt werden, um eine starre Auflagefläche bereitzustellen.

8. Verfahren zum Verbinden einer medizinischen Vorrichtung (10) mit einer Subjektbasisfläche (18), umfassend:
Anordnen eines Pflasterlaminats (22) umfassend eine leitfähige erste Schicht (28) in direkter elektrischer Kommunikation mit der Basisfläche;
elektromechanisches Verbinden des Pflasters mit einer medizinischen Vorrichtungsverbindungsschnittstelle (36), die einen ersten Steckverbinder (46) und Vorrichtungskontakte (50) umfasst, über einen Pflastersteckverbinder (26), wobei der Pflastersteckverbinder elektrische Steckverbinderkontakte (42) in elektrischer Kommunikation mit der ersten leitfähigen Schicht (28) umfasst und einen im Allgemeinen starren Clip (114) mit einer ersten Clipfläche (38) und einer zweiten Clipfläche (40) umfasst, wobei die erste Clipfläche (38) hervorstehende Abschnitte (120) umfasst, wobei ein starrer Pflasterbereich in einer Mitte des Pflasterlaminats (22) vorgesehen ist, wobei das Pflasterlaminat (22) einen flexiblen Außenbereich umfasst,
elektrisches Verbinden einer elektrischen Verteilungsschicht (32) des Pflasterlaminats (22) mit den elektrischen Steckverbinderkontakten (42) und mit jeder von einer Vielzahl von ersten Elektroden, die in der ersten leitfähigen Schicht (28) definiert sind,
wobei der Schritt des Verbindens des Pflasters Folgendes umfasst:
lösbares Einrasten eines Gehäuses (124) der medizinischen Vorrichtung (10) mit den hervorstehenden Abschnitten (120) der ersten Clipfläche (38), um die Steckverbinderkontakte (42) und die Vorrichtungskontakte (50) in elektrischer Kommunikation zu halten, wodurch ein elektrischer Kommunikationspfad zwischen der Subjektbasisfläche (18) und den medizinischen Vorrichtungskontakten (50) hergestellt wird und die medizinische Vorrichtung (10) in unmittelbarer Nähe an dem Pflasterlaminat (22) befestigt wird,
wobei eine Schicht aus einem leitfähigen Z-Achsen-Elastomermaterial (126) komprimiert wird, wenn der Clip (114) auf die medizinische Vorrichtung (10) geschnappt wird, und eine elektrische Verbindung zwischen den medizinischen Vorrichtungskontakten (50) und den elektrischen Steckverbinderkontakten (42) bereitstellt.

9. Verfahren nach Anspruch 8, wobei die Pflasterschichten weiterhin zweite Elektroden (32) umfassen, und wobei das Verfahren weiterhin Folgendes umfasst:
Anordnen der ersten Elektroden in direkter elektrischer Kommunikation mit der Basisfläche und den zweiten Elektroden;
Anordnen der zweiten Elektroden in elektrischer Kommunikation mit den Vorrichtungskontakten; und
Übertragen von elektrischen Signalen, die in der Basisfläche erzeugt werden, an die medizinischen Vorrichtungskontakte.

10. Verfahren nach Anspruch 9, weiterhin umfassend:
Abdichten des elektrischen Kommunikationspfads von der Basisfläche zu den Vorrichtungskontakten gegen Flüssigkeiten mithilfe einer Abdichtungsschicht.

11. Verfahren nach Anspruch 10, wobei der Pflastersteckverbinder elektrische Kontakte umfasst und die Abdichtungsschicht elastische Ringe umfasst, die jeweils jeden jeweiligen einzelnen elektrischen Pflasterkontakt umgeben.

12. Verfahren nach Anspruch 8, wobei der genannte Schritt des Verbindens nach dem genannten Schritt des Anordnens erfolgt.

## Revendications

1. Dispositif modulaire (8) pour une fixation sur la peau (18) d'un patient (12) comprenant :
un dispositif médical (10) qui comporte des contacts électriques du dispositif (50) sur une surface inférieure (52) du dispositif médical (10) ;
un stratifié du patch (22) comportant une pluralité de couches (24), la pluralité de couches (24) comprenant une première couche conductrice (28) pour établir un contact direct avec la peau (18) ;
un connecteur de patch (26) qui comporte des contacts électriques du connecteur (42) en communication électrique avec la première couche conductrice (28) et établit une voie de communication électrique entre la peau (18) et les contacts du dispositif médical (50) et qui fixe le dispositif médical (10) à proximité immédiate du stratifié du patch (22),
dans lequel la pluralité de couches (24) comprend en outre une couche de distribution électrique (32) qui est raccordée électriquement aux contacts électriques du connecteur (42) et à chacune d'une pluralité de premières électrodes définies dans la première couche conductrice (28), et
dans lequel le connecteur de patch comporte une pince généralement rigide (114) comportant une première surface de pince (38) et une deuxième surface de pince (40), la première surface de pince (38) comportant des parties de prolongement (120) qui sont en prise de manière détachable avec un boîtier (124) du dispositif médical (10) pour maintenir les contacts du connecteur (42) et les contacts du dispositif (50) en communication électrique ; en outre le connecteur de patch (26) :
constituant une zone de patch rigide dans un centre du stratifié du patch (22), et une zone externe flexible du stratifié du patch (22), et
comprenant une couche d'un matériau élastomère conducteur dans l'axe Z (126) configurée pour être comprimée lorsque la pince (114) est encliquetée sur le dispositif médical (10) et pour constituer une connexion électrique entre les contacts du dispositif médical (50) et les contacts électriques du connecteur (42).

2. Dispositif selon la revendication 1, dans lequel le connecteur du patch (26) comporte en outre :
des moyens d'alignement (144, 146) pour indexer les contacts électriques du connecteur (42) pour faire concorder les contacts du dispositif médical (50).

3. Dispositif selon la revendication 1, comportant en outre :
un joint fluide (142, 152) qui entoure les contacts du connecteur (42).

4. Dispositif selon la revendication 1, dans lequel les contacts électriques du connecteur (42) comprennent au moins l'un parmi :
des broches (80) ;
des fixations encliquetables (48) ;
des éléments en argent/ chlorure d'argent (154, 162) ; et
des segments élastiques électriquement conducteurs (150, 164).

5. Dispositif selon la revendication 1, dans lequel les contacts électriques du connecteur (42) comportent :
une bande (140) d'un matériau électrique conducteur dans l'axe Z en contact avec la couche de distribution électrique (32).

6. Dispositif selon la revendication 1, comprenant en outre :
un capteur (180) qui détecte au moins un paramètre attribuable à un mouvement de la peau.

7. Dispositif selon la revendication 1, dans lequel la pince (114) passe sous la couche de distribution électrique (32) et les parties de prolongement (120) de celle-ci sont insérées au travers de la couche de distribution électrique (32) pour constituer une surface de support rigide.

8. Procédé de raccordement d'un dispositif médical (10) à une surface de base d'un sujet (18) comprenant :
la disposition d'un stratifié du patch (22) qui comporte une première couche conductrice (28) en communication électrique directe avec la surface de base ;
le raccordement électromécanique du patch à une interface de connexion du dispositif médical (36) qui comporte un premier connecteur (46) et des contacts du dispositif (50) via un connecteur de patch (26), dans lequel le connecteur de patch comporte des contacts électriques du connecteur (42) en communication électrique avec la première couche conductrice (28) et une pince généralement rigide (114) comportant une première surface de pince (38) et une deuxième surface de pince (40), la première surface de pince (38) comportant des parties de prolongement (120), dans lequel une zone de patch rigide est constituée dans un centre du stratifié du patch (22), le stratifié du patch (22) comprenant une zone externe flexible,
le raccordement électrique d'une couche de distribution électrique (32) du stratifié du patch (22) avec les contacts électriques du connecteur (42) et avec chacune d'une pluralité de premières électrodes définies dans la première couche conductrice (28),
l'étape de raccordement du patch comportant :
la mise en prise de manière détachable d'un boîtier (124) du dispositif médical (10) avec les parties de prolongement (120) de la première surface de pince (38) pour maintenir les contacts du connecteur (42) et les contacts du dispositif (50) en communication électrique, établissant ainsi une voie de communication électrique entre la surface de base du sujet (18) et les contacts du dispositif médical (50) et fixant le dispositif médical (10) à proximité immédiate du stratifié du patch (22),
dans lequel une couche d'un matériau élastomère conducteur dans l'axe Z (126) est comprimée lorsque la pince (114) est encliquetée sur le dispositif médical (10) et constitue une connexion électrique entre les contacts du dispositif médical (50) et les contacts électriques du connecteur (42).

9. Procédé selon la revendication 8, dans lequel les couches du patch comportent en outre des deuxièmes électrodes (32) et comportant en outre :
la disposition des premières électrodes en communication électrique directe avec la surface de base et les deuxièmes électrodes ;
la disposition des deuxièmes électrodes en communication électrique avec les contacts du dispositif ; et
la transmission des signaux électriques générés dans la surface de base aux contacts du dispositif médical.

10. Procédé selon la revendication 9, comportant en outre :
l'étanchéification de la voie de communication électrique de la surface de base jusqu'aux contacts du dispositif contre les fluides avec une couche d'étanchéification.

11. Procédé selon la revendication 10, dans lequel le connecteur de patch comporte des contacts électriques et la couche d'étanchéification comporte des anneaux élastiques qui entourent respectivement chaque contact électrique individuel respectif du patch.

12. Procédé selon la revendication 8, dans lequel ladite étape de raccordement survient après ladite étape de disposition.
